# FASCICULE DE BREVET EUROPEEN

(11) **EP 1 829 521 B1**
(45) Date de publication et mention de la délivrance du brevet: **01.07.2015**
(21) Numéro de dépôt: 07300844.3
(22) Date de dépôt: 01.03.2007
(51) Int. Cl.: A61K 6/083, A61K 6/087

(54) **Elément prothétique dentaire antimicrobien et procédé de fabrication**
Antimikrobielles Zahnprothesenelement
Antimicrobial dental prosthetic element

(30) Priorité: 02.03.2006 FR 0650739
(43) Date de publication de la demande: 05.09.2007
(73) Titulaire: Société de Recherches Techniques Dentaires - R.T.D., 38120 Saint Egrève (FR)
(72) Inventeur: Reynaud, Pierre-Luc, 38410, VAULNAVEYS LE HAUT (FR); Chu, Manh-Qyunh, 38120, FONTANIL CORNILLON (FR); Rajon, Cyril, 38320, EYBENS (FR)
(74) Mandataire: Denjean, Eric

(56) Documents cités:
- WO-A-01/08590
- WO-A-96/15759
- WO-A-02/100355
- WO-A-03/105785

## Description

L'invention a pour objet un élément prothétique dentaire réalisé en matériau composite, ledit élément présentant des propriétés antimicrobiennes. L'invention concerne également le procédé de fabrication dudit élément prothétique dentaire.

Par "élément prothétique dentaire", on désigne notamment, mais de façon non limitative, les tenons, les renforts de bridge et les abutments pour implant.

Dans la suite de la description, l'invention est plus particulièrement décrite en relation avec un tenon dentaire en matériau composite.

Les tenons dentaires sont utilisés pour la reconstitution de dents dépulpées. On distingue deux types de tenons, respectivement :
- les tenons métalliques ou céramiques, et
- les tenons composites.

Les tenons métalliques sont en général réalisés en acier inoxydable. Ils ont pour principaux inconvénients d'être sujets à des phénomènes de corrosion. En outre, ils présentent un module d'élasticité transversale différent de celui de la dentine pour conduire, à terme, à une désolidarisation du tenon.

Pour résoudre ces problèmes, on a proposé des tenons fabriqués en matériau composite tel que ceux décrits notamment dans le document EP-A-432 001 du Demandeur. Ces tenons sont en pratique constitués de fibres longues unidirectionnelles de verre ou de carbone noyées dans une matrice de résine thermodurcissable. De manière générale, la proportion de fibres longues, quelque soit la matière choisie, représente de 55 à 70% en volume du tenon, le complément à 100% étant occupé par la matrice.

Pour assurer le maintien du tenon dans la racine, celui-ci doit être collé, puis scellé dans ladite racine. Le praticien comble ensuite les espaces restants au moyen d'une pâte composite. La prothèse ou couronne vient ensuite recouvrir l'ensemble et est maintenue en position par ajout supplémentaire d'un ciment, éventuellement associé à un adhésif. Malgré le soin apporté aux différentes étapes cliniques lors de la reconstitution de la dent, on observe parfois des récidives de caries conduisant au mieux, à un descellement de la prothèse nécessitant un retraitement complet de la racine, au pire, à une extraction de la dent.

Ces récidives de caries sont dues à une infiltration de germes pathogènes, tels que *Streptococcus mutans, Enterococcus faecalis*, voire dans certains cas, *Staphylococcus aureus.* Une telle infiltration peut être due à des pertes d'étanchéité marginale entre la prothèse, la dentine et le matériau de comblement. Des foyers de bactéries peuvent également subsister lorsque le mordançage de la dentine est mal exécuté et laisse apparaître des débris. Les germes peuvent enfin profiter d'obstacles naturels comme des contre-dépouilles, faisant office de réceptacle.

Pour résoudre ce problème, plusieurs solutions ont été développées telles que des adhésifs ou des ciments antimicrobiens, mais également des solutions de mordançage.

Ainsi, le document US-A-5 385 728 décrit une composition acide de mordançage contenant en tant qu'agent antimicrobien, du chlorure de benzalkonium.

Le document US-A-5 968 253 a pour objet un ciment dentaire à base de phosphate de calcium pouvant contenir un agent antimicrobien sous forme d'un antibiotique ou d'un antiamébique.

Le document US-A-6 326 417 concerne une composition dentaire antimicrobienne utilisée comme adhésif, ciment ou pâte composite de reconstitution contenant des composés d'acide salicylique ou de sulfamide, éventuellement sous forme dérivés.

Les documents US-A-5 408 022, US-A-5 494 987, US-A-5 733 949 décrivent des compositions à base de polymères possédant des propriétés antibactériennes après polymérisation, utilisées pour la mise en oeuvre de peau artificielle, cathéter, lentille de contact, mais également produit dentaire, tel que adhésif, résine, composite. L'agent antimicrobien est un monomère polymérisable, dont le groupement actif est à base de sel quaternaire (chlore, brome).

Le document US-A-6 355 704 décrit un adhésif dentaire antimicrobien bi-composants. Un des composants correspond à un monomère polymérisable antimicrobien présentant un groupe cationique sélectionné dans les bases d'ammonium, pyridinium et phosphonium.

Le document US-A-6 924 325 décrit une composition dentaire antimicrobienne pouvant être utilisée comme composition de restauration endodontique, orthodontique ou prothétique. Cette composition contient comme principe actif, une poudre de verre céramique / zinc-argent ou une poudre zéolite à base d'argent.

Le document US-A-6 071 528 décrit un adhésif ciment dentaire antibactérien, dont le principe actif est sélectionné parmi les antibiotiques, les hydroxydes de métaux alcalins, les hydroxydes alcalino-métalliques ou les oxydes alcalino-métalliques.

D'autres types de produits que ceux précités précédemment présentant des propriétés antimicrobiennes ont été développés. Il s'agit notamment de compositions utilisées pour le blanchiment des dents, telles que décrites dans les documents US-A-5 985 249, US-A-6 036 943, US-A-6 309 625, US-A-6 368 576 dans lesquels l'agent antimicrobien est sélectionné parmi la chlorhexidine, les dérivés de benzoate, la tétracycline ou les composés fluorés.

Le document US-A-6 365 130 décrit un chewing-gum contenant des particules céramiques antimicrobiennes à base de cations métalliques.

Le document US-A-6 267 590 se rapporte à un bracket et un fil orthodontique traités par dépôt d'un polymère céramique zéolite d'argent.

Le document US-A-6 113 993 se rapporte à une méthode de traitement d'un implant dentaire, en général métallique, le traitement consistant à appliquer à la surface de l'implant, un agent liant du type phosphate de calcium. Une fois le traitement effectué, il est proposé de tremper les implants dans une solution d'un agent antimicrobien et ce, avant implantation.

Les documents WO 02/100355 et WO 03/105785 décrivent des prépreg traités par un agent antimicrobien. Les prépreg sont des "pré-imprégnés" constitués de fibres de renfort imprégnées dans une matrice de résine. Les prépreg sont livrés au praticien ou au prothésiste tel quel, ces derniers effectuant eux-mêmes la polymérisation du matériau au moment de la mise en bouche. Ces documents mentionnent la possibilité d'utiliser ces prépreg comme tenon. Dans une telle hypothèse, ceux-ci ne seront pas usinés pour présenter une forme définie et précise au sens des éléments prothétiques de la présente invention, puisque c'est le praticien lui même qui donne manuellement la forme finale. Dans le document WO 02/100355, un agent antimicrobien est appliqué à la surface du prépreg. Dans le document WO 03/105785, l'agent antimicrobien est appliqué à l'état liquide dans les pores d'une matrice de résine fabriquée selon les enseignements du document US5846640. Dans la pratique, on prépare une matrice "poreuse", on incorpore le liquide anti microbien dans les pores, puis on "bouche" le volume restant des pores au moyen d'un matériau polymérisable. Le procédé requiert donc de nombreuses étapes.

Le document WO 96/15759 décrit des tenons dentaires contenant, dans la matrice, des oxydes métalliques susceptibles de conférer au tenon une certaine radio-opacité aux rayons X, la teneur en oxyde métallique étant exprimée par rapport au poids de la matrice. Aucune indication n'est donnée concernant les éventuelles propriétés antimicrobiennes de ces oxydes.

Le Demandeur a constaté qu'il était possible de rendre un élément prothétique antimicrobien en introduisant dans sa matrice au moins un agent antimicrobien choisi dans le groupe comprenant l'oxyde de zinc, l'oxyde de cuivre et l'oxyde d'argent sous forme particulaire à raison de 0,05 à 30% en poids de l'élément, en limitant au maximum l'incidence sur les propriétés mécaniques.

En d'autres termes, l'invention a pour objet un élément prothétique dentaire obtenu par usinage d'un profilé en matériau composite comprenant des fibres noyées dans une matrice contenant de la résine. Cet élément prothétique se caractérise en ce que la matrice contient en outre au moins un agent ayant un effet antimicrobien choisi dans le groupe comprenant l'oxyde de zinc, l'oxyde de cuivre et l'oxyde d'argent à une concentration représentant entre 0.05 et 30% en poids de l'élément prothétique, ledit agent étant sous forme pulvérulente.

Au-delà de 30%, les propriétés mécaniques ne sont pas satisfaisantes et l'effet antimicrobien atteint un plateau. Bien entendu, plus la concentration sera faible, meilleures seront les caractéristiques mécaniques. En dessous de 0,05% en poids, l'effet antimicrobien n'est pas observé.

Dans un mode de réalisation particulier, l'élément prothétique est un tenon. En pratique, le tenon comprend des fibres de renfort noyées dans une matrice comprenant de la résine.

Il peut s'agir de fibres de verre, de carbone, de céramique ou encore de quartz. L agent antimicrobien est choisi dans le groupe comprenant l'oxyde de zinc, l'oxyde de cuivre et l'oxyde d'argent.

Afin d'obtenir une répartition plus homogène dans la matrice et de ne pas présenter une contrainte localisée en étant plus volumineux que le diamètre des filaments des fibres de renfort, l'agent antimicrobien se présente sous la forme de particules extrêmement fines de préférence le diamètre moyen D50 (diamètre moyen de 50% des particules) d'une valeur inférieure à 10 µm, avantageusement inférieure à 5 µm, de préférence de l'ordre de 1µm. Dans un autre mode de réalisation, les particules ont une taille nanométrique comprise entre 30 et 80 nm.

Avantageusement, l'agent antimicrobien représente entre 2 et 10% en poids de l'élément prothétique.

Parallèlement et en général, la matrice représente entre 30 et 40% en volume de l'élément prothétique ou 20 et 45 % en poids.

Dans un mode de réalisation avantageux, l'élément prothétique est non seulement anti microbien mais également radio opaque.

Pour rendre le produit visible aux rayons X, l'élément prothétique contient au moins une substance radio-opaque.

L'homme du métier connaît plusieurs manières de rendre le produit radio-opaque.

Dans une première forme de réalisation, au moins une substance radio-opaque est incorporée dans les fibres. Dans ce cas, on peut utiliser des fibres à base d'oxyde de calcium ou encore d'oxyde de zirconium.

Dans une seconde forme de réalisation, la substance radio-opaque est incorporée dans la matrice. Dans certains cas, l'agent anti-microbien et la substance radio-opaque correspondent à un même produit. Cette forme particulière de réalisation présente l'avantage de conserver des propriétés mécaniques et de simplifier la fabrication du profilé. En général, la diminution des caractéristiques mécaniques est proportionnelle à la quantité de charges introduite dans la matrice. Grâce à cette double fonction, il est intéressant de combiner plusieurs agents antimicrobiens afin d'obtenir un matériau antimicrobien à large spectre c'est à dire actif sur les souches de *Streptococcus mutans et d'Enterococcus faecalis* tout en conservant à la fois les propriétés mécaniques et la radio-opacité .

Dans une troisième forme de réalisation, la substance radio-opaque est incorporée à la fois dans la résine et dans les fibres.

En pratique, selon l'invention la substance radio-opaque est choisie dans le groupe comprenant les oxydes métalliques, tels que oxyde d'aluminium, oxyde de baryum, oxyde de strontium, oxyde de tungstène, oxyde de zinc, oxyde de zirconium, les composés fluorés, tels que l'ytterbium et l'yttrium, les carbonates tels que carbonate de lantanium et carbonate de zirconium, seuls ou en mélange.

L'incorporation d'agent antimicrobien s'effectue dans la matrice de résine qui va ensuite imprégner et lier toutes les fibres entre-elles. Le matériau composite ainsi obtenu possèdera au moins un agent antibactérien noyé dans sa masse. Le profilé après usinage peut être un tenon dentaire, un renfort de bridge ou d'abutment pour implant ou de tout élément dentaire préfabriqué.

Lorsque les dispositifs sont des tenons ou renforts de bridge, le praticien se trouve souvent dans l'obligation de découper et fraiser au moins partiellement pour les adapter à l'anatomie. Du fait que la matrice contienne un agent antibactérien, la surface exposée présente toujours une activité antibactérienne.

Les matrices de résine sont des polymères à base de résine époxy, méthacrylate, polyester ou vinylester.

Les fibres de renfort peuvent être des fibres de carbone, de verre, de quartz, de silice.

La fabrication du profilé fait appel à la technique dite de pultrusion connue de l'homme du métier. La section du profilé est variable selon les produits finis à usiner.

Pour obtenir les formes définitives des tenons, renforts de bridge... les techniques d'usinage bien connues de l'homme du métier comme le décolletage, la commande numérique sont employées.

L'invention et les avantages qui en découlent ressortiront mieux des exemples non limitatifs de réalisation suivants.

### Exemple 1

Le profilé de composition ci-dessous est réalisé :
▪ oxyde de zinc pureté 99% taille 1 µm (moyenne D50) : 5% en poids
▪ matrice de résine époxy à base Bisphénol A : 29% en poids
▪ fibres de verre E : 66% en poids

Trois échantillons de diamètre 8 mm issus de ce profilé sont soumis aux essais d'activité antibactérienne en même temps que trois échantillons non traités utilisés comme témoin. La méthode d'essais est inspirée de la norme Japanese Industrial Standard JIS Z 2801 *"Antimicrobial products - Test for antimicrobia activity and efficacy".*

La souche bactérienne employée est le *Staphylococcus aureus.*

Tous les échantillons sont mis en incubation pendant 24h à 38°C. Les populations de *Staphylococcus aureus* sont ensuite décomptées. L'activité antibactérienne est égale au log de la différence entre le nombre de bactéries dénombrées sur l'échantillon témoin moins le nombre de bactéries dénombrées sur l'échantillon à tester. Cette activité antibactérienne est exprimée en puissance log.

Les échantillons issus de cet exemple ont phagocyté pratiquement toutes les bactéries puisque l'on dénombre 2,19 log germes après 24h d'incubation alors que le nombre est de 6,58 log pour les échantillons témoins. La différence entre ces deux moyennes correspond à l'activité bactéricide de l'échantillon à tester.

L'activité bactéricide est de 4,39 log sur *Staphylococcus aureus.*

Le profilé est ensuite usiné pour obtenir des tenons dentaires.

### Exemple 2

Le profilé de composition ci-dessous est réalisé :
▪ oxyde de zinc pureté 99% taille 1 µm (moyenne D50) : 8% en poids
▪ charges de verre contenant l'oxyde de strontium, oxyde de zirconium taille 1 µm (moyenne D90) : 4%
▪ matrice de résine époxy à base Bisphénol A : 30% en poids
▪ fibres de verre type E : 58% en poids

Les échantillons issus de cet exemple sont soumis aux essais d'activité antibactérienne décrite dans l'exemple précédent.

L'activité bactéricide est de 3,49 log sur *Staphylococcus aureus.*

### Exemple 3

Dans cet exemple, on teste le caractère anti microbien de profilés contenant certains oxydes métalliques vis-à-vis d'une souche *Streptococcus mutans.* Les tests sont réalisés selon la norme J15Z2801 "antimicrobial products. Test for antimicrobial activity and efficacity".

Les profilés ont la composition suivante :
▪ oxyde métallique : 5% en poids
▪ matrice de résine époxy à base Bisphénol A : 37% en poids
▪ fibres de verre type E : 58% en poids

| Principe actif | Résultats | Activité (Log) |
|---|---|---|
| Oxyde de magnésium | Inactif | 0.42 |
| Oxyde de cuivre | Bactéricide | 3.94 |
| Oxyde d'argent | Bactéricide | 2.67 |

Ces résultats montrent que pour une concentration très faible, de l'ordre de 5% en poids, l'effet anti microbien est obtenu pour l'oxyde de cuivre et l'oxyde d'argent. A cette concentration, la diminution des caractéristiques mécaniques du tenon reste acceptable.

## Revendications

1. Elément prothétique dentaire obtenu par usinage d'un profilé en matériau composite comprenant des fibres noyées dans une matrice contenant de la résine, **caractérisé en ce que** la matrice contient en outre au moins un agent ayant un effet antimicrobien choisi dans le groupe comprenant l'oxyde de zinc, l'oxyde de cuivre et l'oxyde d'argent à une concentration représentant entre 0.05 et 30% en poids de l'élément prothétique, ledit agent étant sous forme pulvérulente.

2. Elément prothétique selon la revendication 1, **caractérisé en ce que** l'agent antimicrobien représente entre 2 et 10% en poids de l'élément prothétique.

3. Elément prothétique selon l'une des revendications précédentes, **caractérisé en ce que** le diamètre moyen D50 de l'agent anti microbien a une valeur inférieure à 10 µm, avantageusement inférieure à 5 µm, de préférence de l'ordre de 1µm.

4. Elément prothétique selon l'une des revendications 1 à 2, caractérisé, ce que les particules d'agent anti microbien ont une taille nanométrique comprise entre 30 et 80 nm.

5. Elément prothétique selon l'une des revendications précédentes, **caractérisé en ce que** la matrice représente de 20 à 45% en poids de l'élément prothétique.

6. Elément prothétique selon l'une des revendications précédentes, **caractérisé en ce qu'**il s'agit d'un tenon, d'un renfort de bridge, d'un abutment pour implant.

## Patentansprüche

1. Dentalprothetisches Element, das durch Bearbeiten eines Profils aus Verbundstoff erhalten wird, der in eine harzhaltige Matrix eingebettete Fasern umfasst, **dadurch gekennzeichnet, dass** die Matrix darüber hinaus ein Mittel mit einer antimikrobiellen Wirkung enthält, das aus der Gruppe ausgewählt ist, die Zinkoxid, Kupferoxid und Silberoxid in einer Konzentration umfasst, die zwischen 0,05 und 30 Gew.-% des prothetischen Elements darstellt, wobei das Mittel in Pulverform vorliegt.

2. Prothetisches Element nach Anspruch 1, **dadurch gekennzeichnet, dass** das antimikrobielle Mittel zwischen 2 und 10 Gew.-% des prothetischen Elements darstellt.

3. Prothetisches Element nach einem der vorhergehenden Ansprüche, **dadurch gekennzeichnet, dass** der mittlere Durchmesser D50 des antimikrobiellen Mittels einen Wert unter 10 µm, vorteilhafter Weise unter 5 µm, vorzugsweise in der Größenordnung von 1 µm hat.

4. Prothetisches Element nach einem der Ansprüche 1 bis 2, **dadurch gekennzeichnet, dass** die Partikel des antimikrobiellen Mittels eine Nanometergröße haben, die zwischen 30 und 80 nm beträgt.

5. Prothetisches Element nach einem der vorhergehenden Ansprüche, **dadurch gekennzeichnet, dass** die Matrix 20 bis 40 Gew.-% des prothetischen Elements darstellt.

6. Prothetisches Element nach einem der vorhergehenden Ansprüche, **dadurch gekennzeichnet, dass** es sich um einen Stift, eine Brückenverstärkung, ein Implantat-Abutment handelt.

## Claims

1. Prosthetic dental element comprising a machined rod of composite material including fibers embedded in a matrix containing resin, wherein the matrix also contains at least one agent with an antimicrobial effect chosen in the group comprising zinc oxide, copper oxide and silver oxide at a concentration between 0.05 and 30% by weight of the prosthetic element, said agent being in powder form.

2. Prosthetic element according to claim 1, wherein the at least one agent with antimicrobial effect accounts for 2 to 10% by weight of the prosthetic element.

3. Prosthetic element according to one of the preceding claims, wherein a median diameter D50 of the at least one agent with an antimicrobial effect has a value of less than 10 µm, advantageously less than 5 µm, preferably of about 1 µm.

4. Prosthetic element according to claim 1 or 2, wherein particles of at least one agent with an antimicrobial effect have a nanometric size between 30 and 80 nm.

5. Prosthetic element according to one of the preceding claims, wherein matrix accounts for 20 to 45% by weight of the prosthetic element.

6. Prosthetic element according to one of the preceding claims, wherein the prosthetic element is a post, a bridge reinforcement or an implant abutment.
